(19) Europäisches Patentamt
European Patent Office
Office européen des brevets

(11) Publication number: 0 344 791 A2

# (12) EUROPEAN PATENT APPLICATION

(21) Application number: 89109964.0

(22) Date of filing: 01.06.89

(51) Int. Cl.4: C12P 41/00 , C12P 7/62 , //C12N11/00,(C12P7/62, C12R1:38)

(30) Priority: 02.06.88 IT 2083488

(43) Date of publication of application: 06.12.89 Bulletin 89/49

(84) Designated Contracting States: AT BE CH DE ES FR GB LI NL SE

(71) Applicant: Montedison S.p.A.
31, Foro Buonaparte
I-20121 Milan(IT)

(72) Inventor: Bianchi, Daniele, Dr.
50, Via Mosè Bianchi
I-20149 Milano(IT)
Inventor: Cesti, Pietro, Dr.
51, via Torino
I-28069 Trecate Novara(IT)
Inventor: Golini, Paolo, Dr.
4, Via Adua
I-20029 Turbigo Milano(IT)

(74) Representative: Barz, Peter, Dr. et al
Patentanwälte Dipl.-Ing. G. Dannenberg Dr. P. Weinhold, Dr. D. Gudel Dipl.-Ing. S. Schubert, Dr. P. Barz Siegfriedstrasse 8
D-8000 München 40(DE)

## (54) Process for the enzymatic resolution of the optical isomers of racemic ester derivatives of 3-mercapto-2-alkyl-propionic acid.

(57) A process is disclosed for the biotechnological resolution by means of stereoselective enzymatic thio-transesterification of racemic mixtures of compounds of the formula (I):

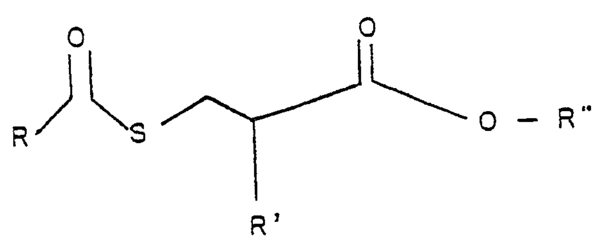

(I)

wherein:
R, R′ and R″, the same or different from each other, represent linear $C_{1-4}$-alkyl groups;
in the presence of an enzyme capable of selectivity causing the thio-transesterification reaction of the (L)-isomer to take place, leaving the (D)-isomer substantially unchanged.

EP 0 344 791 A2

# PROCESS FOR THE ENZYMATIC RESOLUTION OF THE OPTICAL ISOMERS OF RACEMIC ESTER DERIVATIVES OF 3-MERCAPTO-2-ALKYL-PROPIONIC ACID

The present invention relates to a process for the enzymatic resolution of the optical (D)- and (L)-isomers of racemic ester derivatives of 3-mercapto-2-alkyl-propionic acids, particularly 3-mercapto-2-methyl-propionic acid.

More particularly, the present invention relates to a biotechnological process for the separation, or resolution, of the optical isomers of compounds of formula (I):

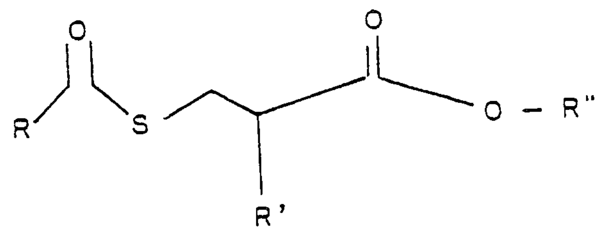

(I)

wherein:
R, R′ and R″, the same or different from each other, represent linear $C_{1-4}$-alkyl groups (particularly methyl and ethyl, but also n-propyl and n-butyl),
carried out in the presence of enzymes endowed with esterase activity and derived from microorganisms.

The compounds or formula (I), in particular the optical (D)-isomers thereof, are an important class of intermediates which may advantageously be used, e.g., in the synthesis of drugs with antihypertensive action (e.g., Captopril).

On the other hand, it is known (D.W. Cushman, H.S. Cheung, E.F. Sabo and M.A. Ondetti, Biochemistry, 16, 5484 (1977)) that the effect of such drugs as inhibitors of angiotensin-converting drugs is strictly related to the configuration of the mercaptoalkanoic chain; in particular, the (D)-isomer is 100 times as active as the corresponding (L)-isomer.

Therefore, there is a great interest in an efficient method for the separation of the optically active isomers, i.e., of the (D)-isomer from the (L)-isomer, of the racemate of the compounds of formula (I) from each other.

Methods for resolving derivatives of 3-mercapto-2-methyl-propionic acid, and in particular of 3-acyl-mercapto-2-methyl-(or -alkyl)-propionic acids are known. Said methods use the formation of diastereoisomeric salts with optically active amines (EP-A-8 833) or the stereoselective enzymatic hydrolysis of corresponding esters (EP-A-130 752 and EP-A-172 614).

Such methods show drawbacks from the viewpoint of their application at an industrial level, in that they use expensive resolving agents, or do not make it possible to obtain products of high optical purity.

Therefore, there is a need of having available a method, suitable for being implemented at an industrial level, which allows to separate from each other the optical isomers of 3-mercapto-2-alkyl-propionic acid esters, as defined above, in a simple, efficient and inexpensive way.

An object of the present invention is,therefore,the provision of a process for the separation, or resolution, of the optical isomers of the derivatives of 3-mercapto-2-alkyl-propionic acids of formula (I) in a simple, efficient and inexpensive way and with a high degree of optical purity.

It has surprisingly been found that this object can be achieved by means of a selective enzymatic thio-transesterification of the racemates of the compounds of formula (I), with the aid of a particular class of enzymes, which will be defined in more detail in the following.

In practice an enzyme is used which belongs to the class of microorganism-derived lipases, capable of causing a thio-transesterification to take place stereoselectively on the (L)-form of the racemates of the compounds of formula (I), while leaving the (D)-isomer substantially unaffected.

Therefore, the present invention is directed to a process for the enzymatic resolution of a racemic mixture of optical isomers of compounds of formula (I):

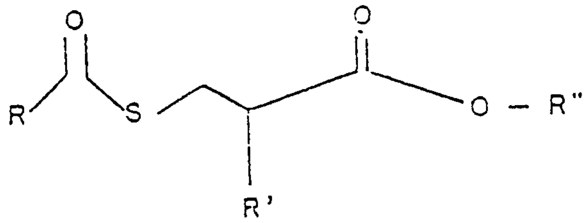

(I)

wherein:

R, R' and R'', the same or different from each other, represent a linear $C_{1-4}$-alkyl group,

which process is characterized in that the racemic mixture of compounds of formula (I) is reacted with an alcohol of formula (II):

R'''-OH (II)

wherein:

R''' represents a linear ($C_1$-$C_8$)-(particularly $C_{1-4}$)-alkyl group,

in the presence of a microorganism-derived lipase, either free or immobilized on a porous support, which lipase is capable of selectively causing the thio-transesterification reaction of the (L)-isomer, while leaving the (D)-isomer of the compounds of formula (I) used as the starting material substantially unchanged, said (D)-isomer being then separated by means of known techniques.

The racemic compounds of formula (I) used as the starting compounds are per se known and can be synthesized according to conventional techniques (see e.g. EP-A-172 614).

According to a schematic representation of the process of the present invention, the racemic esters of formula (I) are reacted, in the presence of an enzyme of the type as defined above, with an alkanol of formula (II) according to the reaction scheme:

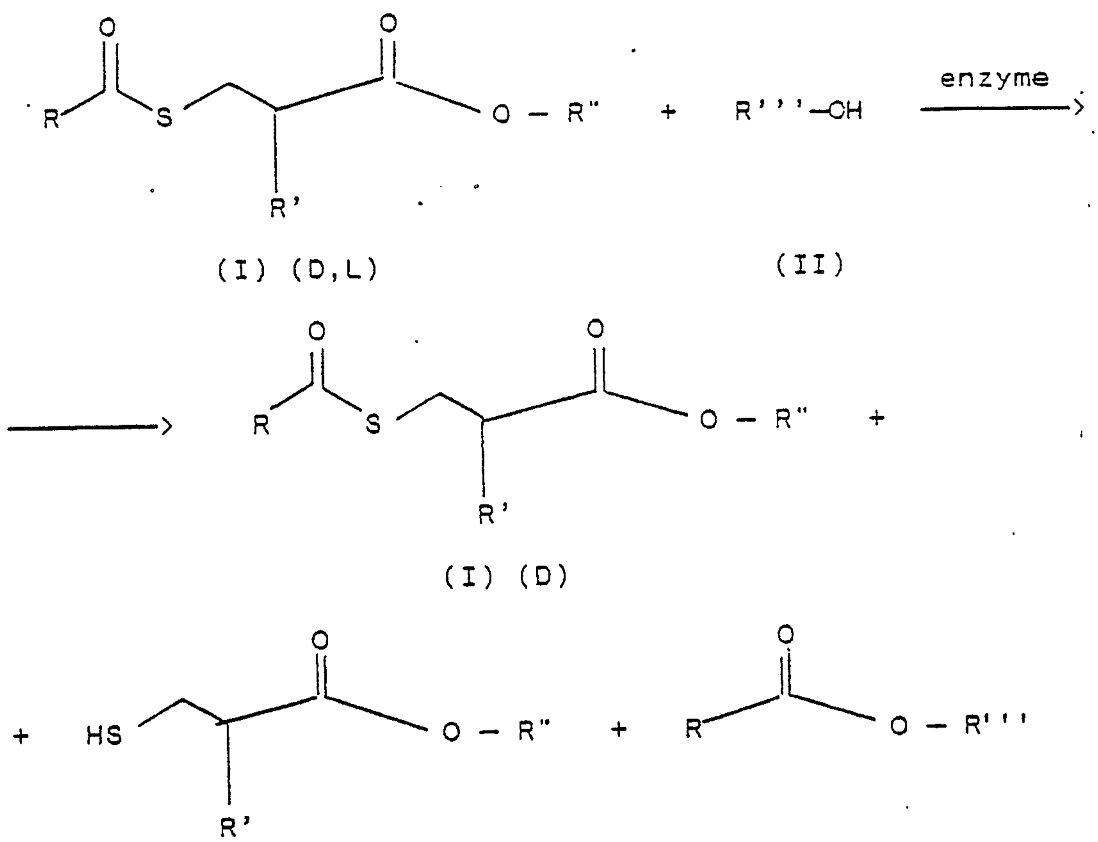


(I) (D,L)

(II)

(I) (D)

(III) (L)

wherein R, R'' and R''' have the above meanings.

Preferred alkanols of formula (II) are ethanol, propanol and butanol.

Said alcohols are preferably used in excess with respect to the substrate of formula (I), being used in the reaction both as reactants and as solvents. Particularly preferred are molar ratios of said alcohols of formula (II) to said esters of formula (I) which are within the range of from 3:1 to about 100:1, particularly from 5:1 to about 10:1.

The weight ratio of the enzyme used to the substrate of formula (I) preferably ranges from 1:5 to about 1:50, particularly from 1:10 to about 1:20.

The thio-transesterification is preferably carried out by vi gorously stirring the reaction mixture comprising the reactant (I), dissolved in the alcohol (II), which simultaneously acts as reactant and solvent, and the enzyme, which is either free or supported, as disclosed below in more detail, at temperatures of from 10° C to 60° C, and particularly of from 20° C to about 30° C.

At the end of the reaction, the enzyme is filtered off; it can be recovered and used again.
From the filtrate, which consists of the organic reaction phase, the ester (I) in the (D)-form and the thiol (III), in the (L)-form, are separated by conventional means such as column chromatography.

As an alternative, the thiol of formula (III) can be extracted from the reaction mixture, after dilution with a solvent immiscible with water (for example, ethyl ether, dichloromethane, etc.), by means of a simple washing with 5% aqueous NaOH.

The optical purity of the esters of formula (I), obtained as described above, was predominantly determined by N.M.R. spectroscopy in the presence of chiral europium complexes.

The absolute configuration of said esters was determined by comparing the data for 3-mercapto-2-alkyl-propionic acid obtained after acidic hydrolysis to the available literature data (M. Shimazaki et al., Chem. Pharm. Bull. 30 (9), 3139, 1982).

The enzymes used in the present invention belong to the class of the lipases of microbial origin.
P Lipase and CES Lipase, both derived from Pseudomonas, (from Pseudomonas fluorescens and from Pseudomonas sp., resp.) and marketed by Amano Pharm. Co., Japan, have proved to be particularly suitable.

According to the present invention, the enzymes can be used either free or immobilized on suitable supports in order to increase their activity and stability, and to facilitate their recovery.

Porous supports having a high surface area such as, e.g., celite, porous glass, Amberlite XAD 7, Amerlite XAD 8, etc. (trade names of Rohm and Haas - U.S.A.) have proved to be particularly suitable for the intended purpose.

The immobilization can easily be carried out by adsorbing a buffered aqueous solution of the enzyme on the support and thereafter evaporating the solvent to dryness.
The following examples illustrate the present invention.
The abbreviation "e.e." means "enantiomeric excess".

## EXAMPLE 1

### Immobilization of Amano P lipase on celite

To 1 g of celite 577 (sold by Johns Manville Ltd., Richmond Surrey), 250 mg of enzyme (Amano P Lipase) dissolved in 5 ml of 0.1 N Na/K phosphate buffer, pH 7, were added.
The resulting mixture was stirred so as to obtain a homogeneous distribution of the enzyme and was then dried in air at 20° C for 18 hours.

### Resolution of the enatiomers of methyl 3-acetyl-thio-2-methyl-propionate

To a solution of 2 g of methyl 3-acetyl-thio-2-methyl-propionate in 6 ml of n-propanol, 120 mg of Amano P Lipase immobilized on 480 mg of celite (see above) were added.

The mixture was vigorously stirred at 20° C and the progress of the reaction was monitored by gas-chromatography.
After 48 hours (57% conversion), the supported enzyme was recovered by filtration and propanol was evaporated under reduced pressure.

The residue was chromatographed on a silica-gel column, using a 9/1 (vol/vol) mixture of hexane/ethyl

ether as the eluent.

Obtained were 790 mg of methyl 3-acetyl-2-D-methyl-propionate as colourless oil with $[a]_D^{20}$ = -60.1°-(C=1, $CHCl_3$), e.e. = 88%, and 850 mg of methyl 3 -mercapto-2-L-methyl-propionate as colourless oil with $[a]_D^{20}$ = +16° (C=1, $CHCl_3$).

## EXAMPLE 2

### Resolution of the enantiomers of methyl 3-acetyl-thio-2-methyl-propionate

The process was carried out under the same conditions as described in Example 1, but using 120 mg of Amano CES Lipase instead of Amano P Lipase.

After 30 hours (conversion of 56%), the supported enzyme was recovered by filtration and the products were separated as described above.

Obtained were 750 mg of methyl 3-acetylthio-2-D-methyl-propionate with $[a]_D^{20}$ = -61.1° (C=1, $CHCl_3$), e.e. = 90% and 860 mg of methyl 3-mercapto-2-L-methyl-propionate with $[a]_D^{20}$ = +15.9° (C=1, $CHCl_3$).

## EXAMPLE 3

### Resolution of the enantiomers of propyl 3-acetyl-thio-2-methylpropionate

The process was carried out under the same conditions as described in Example 1, but using 2 g of propyl 3-acetyl-thio-2-methyl-propionate.

After 48 hours (conversion of 55%), the immobilized enzyme was recovered by filtration.

The alcoholic solution was diluted with 50 ml of ethyl ether and washed with 50 ml of 5% aqueous NaOH.

The organic phase was thoroughly dried over sodium sulphate and thereafter the solvent was evaporated under reduced pressure.

The residue was composed of 88 mg of propyl 3-acetylthio-2-D-methyl-propionate, a colourless liquid with $[a]_D^{20}$ = -48.9° (C=1, $CHCl_3$), i.e. = 92%.

## EXAMPLE 4

### Resolution of the enantiomers of 3-acetyl-thio-2-methyl-propionate

The process was carried out under the same conditions as described in Example 3, but using 7 ml of n-butanol instead of n-propanol.

After 24 hours (conversion of 57%, the supported enzyme was recovered by filtration and the residue was treated as described above.

Obtained were 790 mg of propyl 3-acetylthio-2-D-methyl-propionate as colourless liquid with $[a]_D^{20}$ = -51.8° (C=1, $CHCl_3$), e.e. = 95%.

## Claims

1. Process for the enzymatic resolution of racemic mixtures of compounds of formula (I):

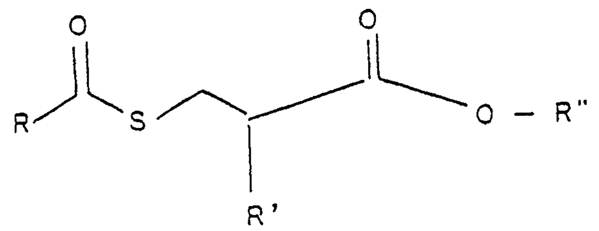

(I)

wherein:
R, R′ and R″, the same or different from each other, represent linear $C_{1-4}$-alkyl groups,
which process is characterized in that a racemic mixture of compounds of formula (I) is reacted with an alcohol of formula (II):
R‴-OH (II)
wherein:
R‴ represents a linear ($C_1$-$C_8$)-alkyl group,
in the presence of a microorganism-derived lipase which is capable of causing selectively the thio-transesterification reaction of the (L)-isomer while leaving the (D)-isomer of the starting mixture substantially unchanged, said (D)-isomer being then separated by means of known techniques.

2. Process according to claim 1, characterized in that the alcohol of formula (II) is selected from ethyl alcohol, propyl alcohol and butyl alcohol.

3. Process according to any one of claims 1 and 2, characterized in that said alcohol of formula (II) is used in excess with respect to the racemic mixture of the compounds of formula (I).

4. Process according to claim 3, characterized in that the molar ratio of said alcohol of formula (II) to said compounds of formula (I) is within the range of from 3:1 to 100:1, particularly of from 5:1 to about 10:1.

5. Process according to any one of claims 1 to 4, characterized in that the weight ratio of the enzyme to said compounds of formula (I) ranges from 1:5 to about 1:50, particularly from 1:10 to about 1:20.

6. Process according to any one of the preceding claims, characterized in that it is carried out at a temperature of from 10 °C to about 60 °C, particularly of from 20 °C to about 30 °C.

7. Process according to any one of the preceding claims, characterized in that said lipase of microbial origin is a lipase derived from Pseudomonas and preferably is selected from P Lipase derived from Pseudomonas fluorescens, and CES Lipase derived from Pseudomonas sp.

8. Process according to any one of the preceding claims, characterized in that said enzyme is used in a free state.

9. Process according to any one of claims 1 to 7, characterized in that said enzyme is used in an immobilized state on a support, particularly a porous support having a high surface area.

10. Process according to claim 9, characterized in that the porous support is selected from celite, porous glass and amberlites.